# EUROPÄISCHE PATENTSCHRIFT

(11) **EP 1 524 004 B1**
(45) Veröffentlichungstag und Bekanntmachung des Hinweises auf die Patenterteilung: **14.02.2018**
(21) Anmeldenummer: 04020827.4
(22) Anmeldetag: 02.09.2004
(51) Int. Cl.: A24F 47/00

(54) **Vorrichtung zur Schalldämpfung bei Beatmungsgeräten**
Noise damping assembly for an inhalation device
Dispositif pour l'insonorisation d'appareil de ventilation

(30) Priorität: 17.10.2003 DE 10348288
(43) Veröffentlichungstag der Anmeldung: 20.04.2005
(73) Patentinhaber: Löwenstein Medical Technology S.A., 2557 Luxembourg (LU)
(72) Erfinder: Franke, Stefan, 22547 Hamburg (DE)
(74) Vertreter: Patentanwälte Klickow & Partner Partnerschaftsgesellschaft mbB

(56) Entgegenhaltungen:
- EP-A- 0 729 762
- EP-A- 1 234 592
- WO-A-99/22793
- WO-A-99/22794
- DE-A- 19 731 355
- DE-U- 20 213 232

## Beschreibung

Die Erfindung betrifft eine Vorrichtung zur Schalldämpfung bei Beatmungsgeräten, die ein Gehäuse mit einer Einströmöffnung und einer Ausströmöffnung aufweist und bei der im Gehäuse ein die Einströmöffnung mit der Ausströmöffnung verbindender Strömungskanal ausgebildet ist, der mindestens bereichsweise von einem geschäumten Material begrenzt ist, wobei entlang des Strömungskanals hintereinander mindestens zwei Dämpfungsräume ausgebildet sind, die von einem Verbindungskanal derart ineinander übergeleitet sind, dass die Dämpfungsräume jeweils mindestens bereichsweise einen größeren Strömungsquerschnitt als der Verbindungskanal aufweisen und dass die Dämpfungsräume mit einem im wesentlichen stetigen Konturverlauf in den Verbindungskanal übergeleitet sind.

Eine derartige Vorrichtung wird beispielsweise in der DE-OS 197 31 355 beschrieben. Der Innenraum einer Dämpfungsbox wird dabei durch Strömungsleitelemente unterteilt, um einen im Hinblick auf die Geräuschdämpfung optimierten Strömungsweg bereitzustellen. Gemäß dem Stand der Technik ist es üblich, in ein metallisches Gehäuse der Dämpfungsbox zur Verbesserung der Dämpfungswirkung Platten aus Schaumstoff einzukleben.

Eine weitere Vorrichtung zur Schalldämpfung wird in der DE-OS 101 07 990 beschrieben. Es werden hier herausnehmbare Dämpfungselemente aus Schaumstoff verwendet, um eine Reinigung sowie Desinfektion zu unterstützen.

Aus der WO 99/22794 A ist bereits eine Vorrichtung zur Schalldämmung bekannt, die für eine Verwendung bei Beatmungsgeräten vorgesehen ist und die ein Gehäuse mit einer Einströmöffnung und einer Ausströmöffnung aufweist. Es wird ein geschäumtes Material verwendet und entlang eines Strömungskanals sind zwei Dämpfungsräume mit einem speziellen Konturverlauf angeordnet.

In der DE 202 213 232 U wird ein Geräusch dämmendes Gehäuse zur Aufnahme einer Turbine erläutert.

Die WO 99/22793 A zeigt einen weiteren Schalldämpfer zur Verwendung im Bereich eines Beatmungsgerätes.

In der EP 07 29 762 A wird ein Inhalationsgerät mit einem integrierten Kompressorschalldämpfer beschrieben.

Die EP 1 234 592 A zeigt ein weiteres Verfahren sowie eine Vorrichtung zur Schalldämmung bei Beatmungsgeräten.

Aufgabe der vorliegenden Erfindung ist es, eine Vorrichtung der einleitend genannten Art derart zu konstruieren, dass eine hohe Schalldämpfung bei geringem zusätzlichem Strömungswiderstand erreicht wird.

Diese Aufgabe wird erfindungsgemäß dadurch gelöst, dass ein Ansaugdämpfer für das Beatmungsgerät ausgebildet ist und wobei eine in lotrechter Richtung obere Begrenzung des ersten Dämpfungsraumes, des Verbindungskanals sowie des zweiten Dämpfungsraumes im Wesentlichen geradlinig und hintereinander verlaufen, so dass eine gerade gemeinsame obere Begrenzung bereitgestellt ist und wobei eine gegenüberliegende gemeinsame untere Begrenzung gerundet konturiert ist und eine Kontur entsprechend einem Wandungsabschnitt eines elypsoidartigen Körpers ausbildet.

Durch die Anordnung von mindestens zwei Dämpfungsräumen hintereinander sowie die Verbindung der Dämpfungsräume durch einen Verbindungskanal mit im wesentlichen stetiger Überleitung der jeweiligen Begrenzungskonturen wird ein sehr geringer Strömungswiderstand bei hoher Strömungsgeschwindigkeit erreicht. Darüber hinaus kann eine erhebliche Senkung des breitbandigen Schallpegels der auftretenden Geräusche erreicht werden. Die Dämpfung erfolgt insbesondere im Hinblick auf höhere Frequenzanteile, so daß das verbleibende Restgeräusch im Bereich von subjektiv als angenehmer empfundener tieferen Frequenzen liegt und eine helle Klangfarbe herausgefiltert wird. Trotz der hohen Dämpfungswirkung kann ein kleines Bauvolumen realisiert werden.

Zu einer Realisierung einer kompakten Ausbildung trägt es bei, daß die Einströmöffnung in einen Einströmkanal einmündet, dessen Längsachse im wesentlichen quer zu einer Längsachse des Verbindungskanals orientiert ist.

Ebenfalls wird eine raumsparende Konstruktion dadurch unterstütz, daß die Ausströmöffnung in einen Ausströmkanal einmündet, dessen Längsachse im wesentlichen quer zur Längsachse des Verbindungskanals orientiert ist.

Ein günstiges Strömungsverhalten mit geringem Strömungswiderstand wird dadurch unterstützt, daß der Verbindungskanal im Bereich einer Begrenzungsseite im wesentlichen geradlinig und im Bereich einer zu dieser Seite gegenüberliegenden Begrenzungsseite im wesentlichen gerundet konturiert begrenzt ist.

Eine typische Ausbildung wird dadurch bereitgestellt, daß ein Ansaugdämpfer für das Beatmungsgerät ausgebildet ist.

Zur Erleichterung einer Herstellung des geschäumten Materials wird vorgeschlagen, daß das geschäumte Material ein Schaumstoffteil ausbildet, das in drei Schaumstoffplatten unterteilt ist.

Zur Erleichterung einer Herstellung des geschäumten Materials wird vorgeschlagen, daß das Gehäuse in das Beatmungsgerät eingebaut ist.

Eine einfache Handhabung wird dadurch unterstützt, daß das Gehäuse neben dem Beatmungsgerät angeordnet ist.

Zur Unterstützung einer geringen Strömungsgeschwindigkeit wird vorgeschlagen, daß ein den Einströmkanal in den ersten Dämpfungsraum überleitender Eingangsbereich weit ausgebildet ist.

Ebenfalls trägt es zu einer geringen Strömungsgeschwindigkeit bei, daß ein den Ausströmkanal in den zweiten Dämpfungsraum überleitender Ausgangsbereich weit ausgebildet ist.

In der Zeichnung sind Ausführungsbeispiele der Erfindung schematisch dargestellt. Es zeigen:
- Fig. 1: eine perspektivische sowie teilweise geschnittene Darstellung der Vorrichtung zur Schalldämpfung,
- Fig. 2: eine Seitenansicht gemäß Blickrichtung II in Fig. 1,
- Fig. 3: eine Seitenansicht gemäß Blickrichtung III in Fig.1,
- Fig. 4: einen Längsschnitt gemäß Schnittlinie IV - IV in Fig. 3 und
- Fig. 5: ein Querschnitt gemäß Schnittlinie V - V in Fig. 2.

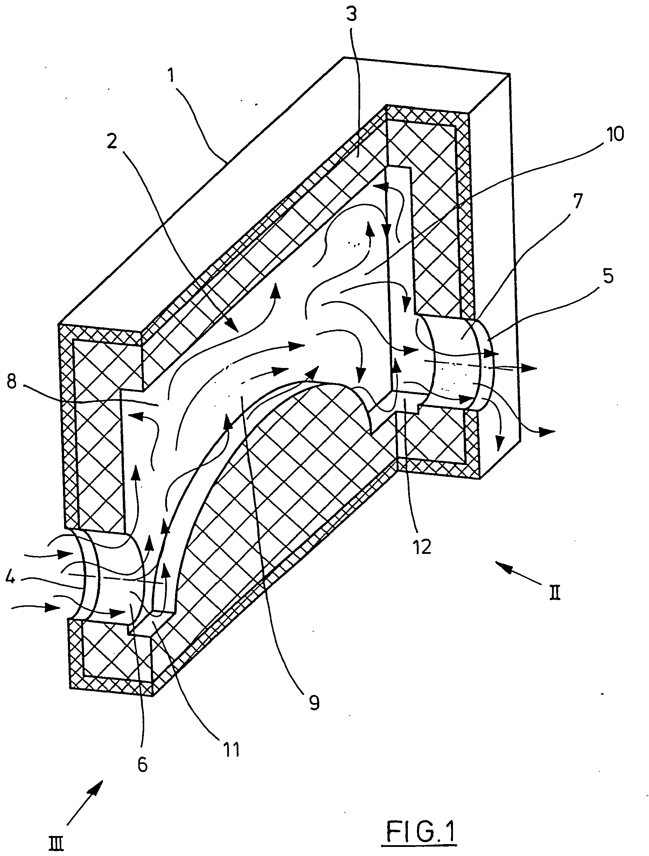

Fig. 1 veranschaulicht den grundsätzlichen Aufbau der Vorrichtung zur Schalldämpfung in einer teilweise geschnittenen Darstellung. In ein Gehäuse (1) ist zur Begrenzung eines Strömungskanals (2) ein Schaumstoffteil (3) eingesetzt. Das Gehäuse (1) weist eine Einströmöffnung (4) sowie eine Ausströmöffnung (5) auf. Die Einströmöffnung (4) mündet in einen Einströmkanal (6) und die Ausströmöffnung (5) in einen Ausströmkanal (7) ein. Der Einströmkanal (6) und der Ausströmkanal (7) bilden jeweils Bereiche des Strömungskanals (2) aus. Im Anschluß an den Einströmkanal (6) ist ein erster Dämpfungsraum (8) angeordnet. Der erste Dämpfungsraum (8) ist von einem Verbindungskanal (9) in einen zweiten Dämpfungsraum (10) übergeleitet. Der Dämpfungsraum (10) mündet in den Ausströmkanal (7) ein.

Das Schaumstoffteil (3) kann beispielsweise aus drei Schaumplatten aufgebaut sein, die in einer SandwichBauweise zusammengesetzt sind. Der erste Dämpfungsraum (8) und der zweite Dämpfungsraum (10) weisen relativ zum Verbindungskanal (9) eine große Strömungsquerschnittfläche auf. Hierdurch werden im Bereich des ersten Dämpfungsraumes (8) ein weiter Eingangsbereich (11) und im Bereich des zweiten Dämpfungsraumes (10) ein weiter Ausgangsbereich (12) bereitgestellt. Eine Überleitung des ersten Dämpfungsraumes (8) in den zweiten Dämpfungsraum (10) erfolgt zumindest im wesentlichen stetig, so daß eine gleichmäßige Strömung unterstützt wird.

Bei dem in Fig. 1 dargestellten Ausführungsbeispiel verlaufen eine in lotrechter Richtung obere Begrenzung des ersten Dämpfungsraumes (8), des Verbindungskanals (9) sowie des zweiten Dämpfungsraumes (10) im wesentlichen geradlinig und hintereinander, so daß eine gerade gemeinsame obere Begrenzung bereitgestellt ist. Eine gegenüberliegende gemeinsame untere Begrenzung ist gerundet konturiert und bildet eine Kontur entsprechend einem Wandungsabschnitt eines elypsoidartigen Körpers aus.

Bei dem in Fig. 1 dargestellten Ausführungsbeispiel erstrecken sich der Einströmkanal (6) und der Ausströmkanal (7) mit ihren jeweiligen Längsachsen quer zu einer Strömungsrichtung des Verbindungskanals (9). Grundsätzlich ist aber auch eine im wesentlichen gemeinsame Orientierung der jeweiligen Längsachsen realisierbar.

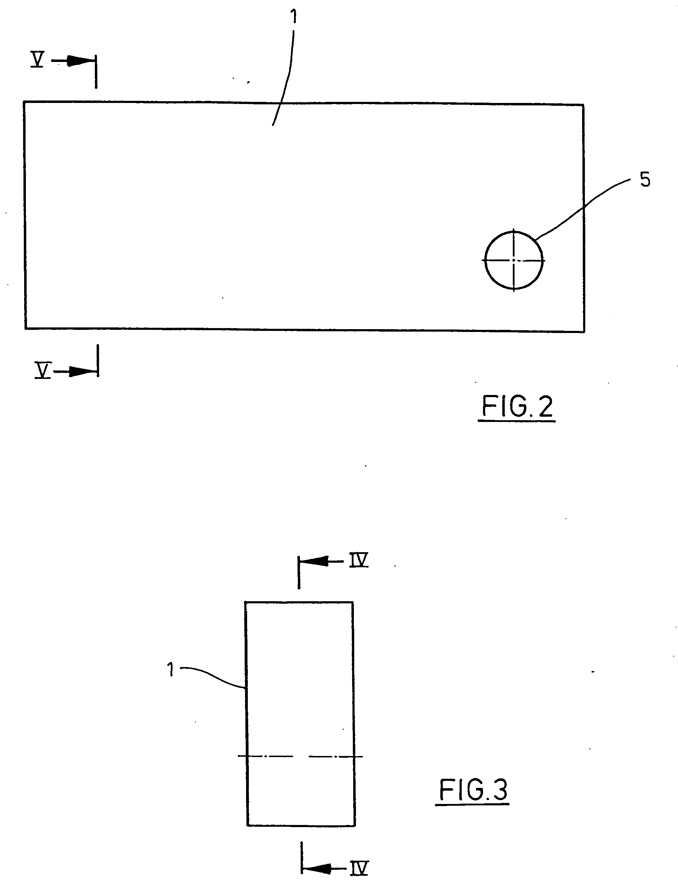

Aus der Seitenansicht in Fig. 2 ist erkennbar, daß das Gehäuse (1) eine im wesentlichen rechteckförmige Außenkontur aufweist. Die rechteckförmige Außenkontur ist nochmals in Fig. 3 veranschaulicht.

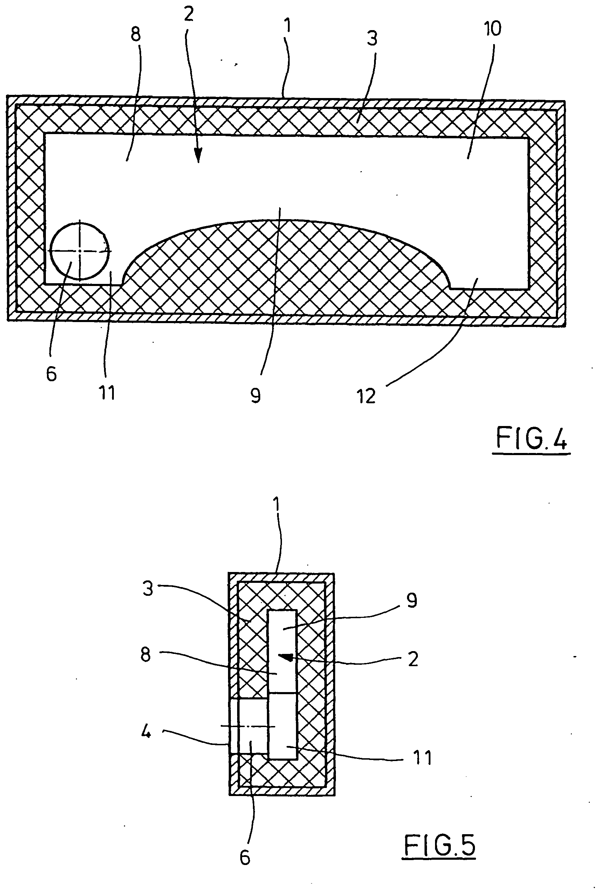

Auf der Längsschnittdarstellung in Fig. 4 ist noch einmal die Ausbildung des Gehäuses (1) sowie des Schaumstoffteils (3) zu erkennen. Die Querschnittdarstellung in Fig. 5 veranschaulicht insbesondere noch einmal die weite Dimensionierung des Ausgangsbereiches (12).

Durch die weite Ausbildung des Eingangsbereiches (11) wird erreicht, daß bereits bei einem Einströmen des Gases in das Dämpfungselement die Strömungsgeschwindigkeit deutlich reduziert wird. Verbleibende Strömungsgeräusche werden überwiegend vom Material des Schaumstoffteils (3) absorbiert. Es wird hierbei ausgenutzt, daß sich Schallwellen im wesentlichen kugelförmig ausbreiten und hierdurch von einem Akustikschaum gut absorbiert werden können. Durch die sanfte Überleitung des ersten Dämpfungsraumes in den zweiten Dämpfungsraum (10) unter Verwendung des Verbindungskanals (9) werden Turbulenzen vermieden, die zu einer Schallemission beitragen bzw. den Strömungswiderstand erhöhen. Die Schalldämpfungswirkung wird durch die partielle Erhöhung der Strömungsgeschwindigkeit im Bereich des Verbindungskanals (9) sowie die erneute Herabsetzung der Strömungsgeschwindigkeit im zweiten Dämpfungsraum (10) nochmals verbessert. Durch die weite Dimensionierung des Ausgangsbereiches (12) tritt die Gasströmung mit einer geringen Strömungsgeschwindigkeit und somit sowohl geräuscharm als auch mit einem geringen Strömungswiderstand in die Ausströmöffnung (5) ein.

Aufgrund der kompakten Gestaltung der Dämpfungsvorrichtung kann diese sowohl als separates Bauteil verwendet als auch innerhalb eines Beatmungsgerätes angeordnet werden. Die jeweilige konkrete Ausbildung kann in Abhängigkeit von den jeweiligen Anwendungsanforderungen erfolgen.

## Patentansprüche

1. Vorrichtung zur Schalldämpfung bei Beatmungsgeräten, die ein Gehäuse mit einer Einströmöffnung und einer Ausströmöffnung aufweist und bei der im Gehäuse ein die Einströmöffnung mit der Ausströmöffnung verbindender Strömungskanal ausgebildet ist, der mindestens bereichsweise von einem geschäumten Material begrenzt ist, wobei entlang des Strömungskanals (2) hintereinander mindestens zwei Dämpfungsräume (8, 10) ausgebildet sind, die von einem Verbindungskanal (9) derart ineinander übergeleitet sind, dass die Dämpfungsräume (8, 10) jeweils mindestens bereichsweise einen größeren Strömungsquerschnitt als der Verbindungskanal (9) aufweisen und dass die Dämpfungsräume (8, 10) mit einem im wesentlichen stetigen Konturverlauf in den Verbindungskanal (9) übergeleitet sind, **dadurch gekennzeichnet, dass** ein Ansaugdämpfer für das Beatmungsgerät ausgebildet ist und wobei eine in lotrechter Richtung obere Begrenzung des ersten Dämpfungsraumes (8), des Verbindungskanals (9) sowie des zweiten Dämpfungsraumes (10) im Wesentlichen geradlinig und hintereinander verlaufen, so dass eine gerade gemeinsame obere Begrenzung bereitgestellt ist und wobei eine gegenüberliegende gemeinsame untere Begrenzung gerundet konturiert ist und eine Kontur entsprechend einem Wandungsabschnitt eines ellipsoidartigen Körpers ausbildet.

2. Vorrichtung nach Anspruch 1, **dadurch gekennzeichnet, dass** die Einströmöffnung (4) in einen Einströmkanal (6) einmündet, dessen Längsachse im Wesentlichen quer zu einer Längsachse des Verbindungskanals (9) orientiert ist.

3. Vorrichtung nach Anspruch 1 oder 2. **dadurch gekennzeichnet, dass** die Ausströmöffnung in einen Ausströmkanal (7) einmündet, dessen Längsachse im Wesentlichen quer zur Längsachse des Verbindungskanals (9) orientiert ist.

4. Vorrichtung nach einem der Ansprüche 1 bis 3, **dadurch gekennzeichnet, dass** das geschäumte Material ein Schaumstoffteil ausbildet, dass in drei Schaumstoffplatten unterteilt ist.

5. Vorrichtung nach einem der Ansprüche 1 bis 4, **dadurch gekennzeichnet, dass** das Gehäuse (1) in das Beatmungsgerät eingebaut ist.

6. Vorrichtung nach einem der Ansprüche 1 bis 4, **dadurch gekennzeichnet, dass** das Gehäuse (1) neben dem Beatmungsgerät angeordnet ist.

7. Vorrichtung nach einem der Ansprüche 1 bis 6, **dadurch gekennzeichnet, dass** ein den Einströmkanal (6) in den ersten Dämpfungsraum (8) überleitender Eingangsbereich (11) weit ausgebildet ist.

8. Vorrichtung nach einem der Ansprüche 1 bis 7, **dadurch gekennzeichnet, dass** ein den Ausströmkanal (7) in den zweiten Dämpfungsraum (10) überleitender Ausgangsbereich (12) weit ausgebildet ist.

## Claims

1. Noise damping assembly for an inhalation device, which comprises a housing with an inflow opening and an outflow opening and in which a flow channel is formed in the housing, which connects the inflow opening to the outflow opening and is bounded at least in some regions by a foamed material, wherein at least two damping spaces (8, 10) are formed one behind the other along the flow channel (2), said damping spaces being merged into one another by a connecting channel (9) such that the damping spaces (8, 10) each have at least in some regions a larger flow cross-section than the connecting channel (9) and such that the damping spaces (8, 10) are merged into the connecting channel (9) with a substantially continuous contour, **characterized in that** an aspiration damper is formed for the inhalation device, and wherein an upper boundary, in the vertical direction, of the first damping space (8), of the connecting channel (9) and of the second damping space (10) run substantially in a straight line and one behind the other so that a straight common upper boundary is provided, and wherein an opposite common lower boundary is contoured in a rounded manner and forms a contour corresponding to a wall section of an ellipsoidal body.

2. Assembly according to claim 1, **characterized in that** the inflow opening (4) opens into an inflow channel (6), the longitudinal axis of which is oriented substantially transversely to a longitudinal axis of the connecting channel (9).

3. Assembly according to claim 1 or 2, **characterized in that** the outflow opening opens into an outflow channel (7), the longitudinal axis of which is oriented substantially transversely to the longitudinal axis of the connecting channel (9).

4. Assembly according to any of claims 1 to 3, **characterized in that** the foamed material forms a foam part which is divided into three foam panels.

5. Assembly according to any of claims 1 to 4, **characterized in that** the housing (1) is built into the inhalation device.

6. Assembly according to any of claims 1 to 4, **characterized in that** the housing (1) is arranged next to the inhalation device.

7. Assembly according to any of claims 1 to 6, **characterized in that** an inlet region (11) which merges the inflow channel (6) into the first damping space (8) is wide.

8. Assembly according to any of claims 1 to 7, **characterized in that** an outlet region (12) which merges the outflow channel (7) into the second damping space (10) is wide.

## Revendications

1. Dispositif d'insonorisation pour appareils de ventilation, qui présente un logement avec une ouverture d'admission et une ouverture d'évacuation et pour lequel dans le logement, se trouve un canal d'écoulement reliant l'ouverture d'admission à l'ouverture d'évacuation, lequel canal d'écoulement est limité au moins localement, par un matériau moussé et où le long du canal d'écoulement (2), au moins deux chambres d'insonorisation (8, 10) sont agencées successivement, un canal de liaison (9) passant d'une chambre d'insonorisation à l'autre, de sorte que les chambres d'insonorisation (8, 10) présentent au moins localement, une coupe transversale plus élevée que le canal de liaison (9) et que le transfert du canal de liaison (9) aux chambres d'insonorisation (8, 10) suit un contour essentiellement continu, **caractérisé en ce que** l'appareil de ventilation présente un silencieux d'aspiration et où la limite supérieure en direction verticale de la chambre d'insonorisation (8), du canal de liaison (9) ainsi que de la deuxième chambre d'insonorisation (10) est sensiblement rectiligne et consécutive, de sorte que l'on obtienne une limite supérieure commune rectiligne et où la limite inférieure commune opposée présente un contour arrondi, le contour correspondant à une section d'un corps éllipsoïde.

2. Dispositif selon la revendication 1, **caractérisé en ce que** l'ouverture d'admission (4) débouche dans un canal d'admission (6), dont l'axe longitudinal est orienté de manière essentiellement transversale à l'axe longitudinal du canal de liaison (9).

3. Dispositif selon la revendication 1 ou 2, **caractérisé en ce que** l'ouverture d'évacuation débouche dans un canal d'évacuation (7), dont l'axe longitudinal est orienté de manière essentiellement transversale à l'axe longitudinal du canal de liaison (9).

4. Dispositif selon l'une des revendications 1 à 3, **caractérisé en ce que** le matériau moussé compose une partie en mousse, qui est divisée en trois plaques de mousse.

5. Dispositif selon l'une des revendications 1 à 4, **caractérisé en ce que** le logement (1) est intégré dans l'appareil de ventilation.

6. Dispositif selon l'une des revendications 1 à 4, **caractérisé en ce que** le logement (1) est agencé à côté de l'appareil de ventilation.

7. Dispositif selon l'une des revendications 1 à 6, **caractérisé en ce qu'**une zone d'entrée (11) faisant le transfert du canal d'admission (6) à la première chambre d'insonorisation (8) est large.

8. Dispositif selon l'une des revendications 1 à 7, **caractérisé en ce qu'**une zone de sortie (12) faisant le transfert du canal d'évacuation (7) à la deuxième chambre d'insonorisation (12) est large.
